Europäisches Patentamt

European Patent Office

Office européen des brevets

(11) Veröffentlichungsnummer: **0 135 071**
**B1**

(12) **EUROPÄISCHE PATENTSCHRIFT**

(45) Veröffentlichungstag der Patentschrift: 16.05.90

(21) Anmeldenummer: 84108906.3

(22) Anmeldetag: 27.07.84

(51) Int. Cl.⁵: **G 01 N 33/533,**
G 01 N 33/543

(54) **Lumineszenz Immunoassay für Haptene und hierfür verwendbares chemilumineszent-markiertes Haptenkonjugat sowie Verfahren zur Herstellung derselben.**

(30) Priorität: 29.07.83 DE 3327327

(43) Veröffentlichungstag der Anmeldung:
27.03.85 Patentblatt 85/13

(45) Bekanntmachung des Hinweises auf die
Patenterteilung:
16.05.90 Patentblatt 90/20

(84) Benannte Vertragsstaaten:
AT BE CH DE FR GB IT LI LU NL SE

(56) Entgegenhaltungen:
EP-A-0 003 583
EP-A-0 047 459
EP-A-0 087 564
US-A-4 169 137
US-A-4 261 893
US-A-4 275 160

(73) Patentinhaber: Henning Berlin GmbH Chemie
und Pharmawerk
Komturstrasse 19-20
D-1000 Berlin 42 (DE)

(72) Erfinder: **Gadow, André**
Lettberger Strasse 43
D-1000 Berlin 47 (DE)
Erfinder: **Wood, W. Graham, Ph.D.**
Am Waldrand 29
D-2401 Gross-Grönau (DE)

(74) Vertreter: **Werner, Hans-Karsten, Dr. et al**
Deichmannhaus am Hauptbahnhof
D-5000 Köln 1 (DE)

# EP 0 135 071 B1

**Beschreibung**

Gegenstand der vorliegenden Erfindung ist ein Lumineszenz Immunoassay für Haptene, die hierin enthaltenen chemilumineszent-markierten Haptenkonjugate sowie Verfahren zu deren Herstellung.

Lumineszenz Immunoassays für Haptene bestehend aus A) einem für das jeweilige Hapten spezifischen Antikörper und B) einem chemilumineszent-markierten Haptenkonjugat. Dieses Haptenkonjugat enthält im allgemeinen eine zur Chemilumineszenz befähigte Gruppe, eine Verknüpfungsgruppe und eine Hapten. Die Verknüpfungsgruppe kann im einfachsten Fall ersetzt werden durch eine direkte chemische Bindung, jedoch ist es üblich, durch eine Verknüpfungsgruppe einen gewissen räumlichen Abstand zu erzielen. Die Verknüpfungsgruppe wird deshalb auch meist "Spacer" genannt. Es hat sich gezeigt, daß bei einer direkten Kopplung von Haptenen mit einer zur Chemiluminszenz befähigten Gruppe die Eigenschaften beider Gruppen so verändert wurden, daß die Empfindlichkeit und Spezifität der Teste verringert wurden. Zum einen veränderten sich die chemilumineszenten Eigenschaften und zum anderen die Spezifität des Haptens zu seinem Antikörper.

Typische chemilumineszent-markierte Konjugate und diese enthaltende Lumineszenz Immunoassays sind beispielsweise aus der DE—OS 29 21 781 bekannt. Die Verknüpfungsgruppe R (dort auch Brückengruppe genannt) soll demnach maximal 1 bis 50, vorzugsweise 1 bis 10 Kohlenstoffatome oder Heteroatome aufweisen, so daß das Molekulargewicht dieser Gruppe 1000 nicht übersteigt und vorzugsweise weniger als 200 beträgt; vgl. S. 34 und 35. Die dort beschriebenen Lumineszenz Immunoassays weisen zwar gegenüber Radioimmunoassays den Vorteil auf, daß man nicht mit radioaktiven Substanzen arbeiten muß, die nur eine begrenzte Lebensdauer haben und wegen der entsprechenden Schutzvorschriften begrenzt eingesetzt werden können, dafür erreichen sie aber bei weiten nicht die von Radioimmunoassays bekannte Empfindlichkeit bzw. Reproduzierbarkeit.

Aus der DE—OS 29 13 549 sind chemisch induzierte Fluoreszenz-Immunteste bekannt, bei welchen der Antiligand spezifisch am epitopen Zentrum des Liganden gebunden wird und als Marker ein lichtemittierendes reziprokes Paar vorgesehen ist, welches aus einer Chemilumineszenzquelle und einem Quencher besteht, der das von der Chemilumineszenzquelle emittierte Licht kollisionsfrei auslöschen kann. Dabei werden Konjugate mit Chemilumineszenzmarkierung und Konjugate mit Quenchermarkierung gebildet und an Bestandteile des immunologischen Paars gebunden. Das darin beschriebene Prinzip beruht auf der Feststellung, daß bei Vorhandensein eines Farbstoffes innerhalb eines begrenzten Abstands von einer chemilumineszierenden Verbindung oder Gruppe im erregten Zustand, die chemilumineszierende Verbindung oder Gruppe ihre Energie auf den Quencher stoßfrei und ohne Strahlungsemission übertragen kann. Der Quencher kann sodann die Strahlung bei einer höheren Wellenlänge als die chemilumineszierende Verbindung oder Gruppe emittieren und kann die Energie durch strahlungslosen Zerfall verlieren. Dabei ist es prinzipiell möglich an höher molekulare Liganden sowohl mehrere Mole chemilumineszierende Substanz oder Quencher zu koppeln. In der Beschreibung wird erwähnt, daß es prinzipiell möglich sei, auch eine Mehrzahl von Markern an den Bestandteil des immunologischen Paares zu binden, wobei sogenannte polyligandenanaloge Marker entstehen. Besondere Vorteile derartiger Systeme sind jedoch nicht erwähnt. Ein Nachteil besteht darin, daß das analoge Paar den Quencher in vergleichbaren Abständen enthalten muß, um entsprechend wirksam zu sein. Bei den relativ kleinen Haptenen ist die Besonderheit zu beachten, daß diese oftmals zu einer wesentlich verrigerten Chemilumineszenz führen, ohne daß zusätzlich ein ausdrücklicher Quencher an den Rezeptor gebunden ist. Diese Wechselwirkung zwischen Hapten und chemilumineszierender Gruppe ist bereits mehrfach beobachtet worden und führt zu verringerter Empfindlichkeit und Spezifität der Teste, weshalb man in solchen Fällen vorzugsweise mit einem "Spacer" arbeitet.

Aus EP—A—0 047 459 ist ein Fluoreszenzreagenz für die Immunfluoreszenz bekannt, welches an einer hydrophilen Zwischenverbindung ein Molekül hydrophobes Hapten und ein hydrophobes, fluoreszierendes Molekül trägt. In EP—A—0 003 583 sind konjugate für den cytochemischen Nachweis von Steroidrezeptoren beschrieben bei denen an das Carrierprotein mehrere Hormonmoleküle und Fluoreszenzmoleküle gebunden sind.

Die vorliegende Erfindung hat sich die Aufgabe gestellt, Lumineszenz Immunoassays für Haptene zu verbessern und sie bezügliche der Handhabung, der Empfindlichkeit und der Reproduzierbarkeit den Radioimmunoassays vergleichbarer zu machen. Dabei soll gleichzeitig angestrebt werden, die enzelnen Bestandteile dieses Immunoassays einfach, reproduzierbar und im größeren Maßstab herstellbar zu machen. Diese Aufgabe wurde überraschenderweise dadurch gelöst, daß man ein chemilumineszent-markiertes Haptenkonjugat verwendet, welches zwischen der zur Chemilumineszenz befähigten Gruppe und dem Hapten eine Verknüpfungsgruppe aufweist, die ein kettenförmigen Polymeres mit wiederkehrenden funktionellen Gruppen ist, an welches pro Mol sowohl mehrere Mole zur Lumineszenz befähigte Gruppen als auch mehrere Mole Hapten gebunden sind.

Dieses Ergebnis war nicht vorhersehbar, da nach dem Stand der Technik die Verknüpfungsgruppe so klein wie mölich gehalten werden sollte und stets nur eine zur Chemilumineszenz befähigte Gruppe mit einem Hapten verbunden werden sollte. Überraschenderweise hat sich gezeigt, daß die Empfindlichkeit und Reproduzierbarkeit eines Chemilumineszenz Immunoassays für Haptene erheblich gesteigert werden kann, wenn man stattdessen mehrere Mole der zur Chemilumineszenz befähigten Gruppe mit mehreren Molen des Haptens in der Weise miteinander verknüpft, daß sie mit ausreichendem Abstand an ein

2

kettenförmiges Polymeres gebunden sind.

Für den erfindungsgemäßen Lumineszenz Immunoassay wird als weitere Komponente ein für das Hapten spezifischer Antikörper verwendet, der vorzugsweise gewonnen wird durch Verwendung eines anderen kettenförmigen Polymeren an welchem durch eine andere chemische Reaktion mehrere Mole Hapten gebunden sind. Die so erhaltenen Antikörper sind hochspezifisch bezüglich des Haptens in freier Form als auch in gebundener Form an ein kettenförmiges Polymeres. Diese Antikörper weisen hingegen keine spezifische Bindungsfähigkeit auf für andere kettenförmige Polymere, an die mit einer anderen chemischen Reaktion Gruppen angekoppelt wurden.

Als kettenförmige Polymere mit wiederkehrenden funktionellen Gruppen können insbesondere Peptide, Glykoproteine, Glykolipide oder Kohlenhydrate eingesetzt werden. Typische Beispiele für derartige Polymere sind Polysaccharide wie Dextrane, Pectine, Lectine und natürliche Gummen, Peptide wie p-Lys, p-Lys-Glu, p-Lys-Tyr, p-Glu-Tyr, Proteine wie Serum-Albumine und Globuline, Glykoproteine wie Transferrin, Thyreoglobulin, Orosomucoid usw.

Die Polymeren müssen eine ausreichende Anzahl wiederkehrender funktioneller reaktiver Gruppen aufweisen, wie Amino-, Carboxyl-, Carbonyl-, Thionyl-, Hydroxyl-, und/oder zur Diazokupplung befähigte Gruppen, an die mit Hilfe üblicher und bekannter Methoden sowohl zur Chemilumineszenz befähigten Gruppen als auch die Haptene an das Polymere gekoppelt werden können.

Derartige Kopplungsreagenzien sind beispielsweise für die direkte Kopplung Glutardialdehyd, Bromcyan, Hydrazine, Bisepoxirane, Divinylsulfone, Epichlorhydrin, Benzochinone, Perjodat, Trichloro-s-Triazine, Isothiocyanate, Arylamine und Phenylhydrazine. Die dabei entstehenden Bindungstypen sind beispielsweise Michael Addukte und Schiff'sche Basen, Cyanat-Ester, Triazinyle, Ether, Imidocarbonate, Amide, gemischte Anhydride, Alkylamine, Ester. Für die indirekte Kopplung kommen Reagenzien in Frage wie EEDQ (N-Ethoxycarbonyl-2-ethoxy-1,2-dihydrochinolin) Carbodiimide wie Dicyclohexylcarbodiimid, 1-Cyclohexyl-3-(2-morpholinyl-(4)-ethyl) carbodiimid-methyl-p-toluol-sulfonat, 1-Ethyl-3-(3-dimethyl-aminopropyl) carbodiimid hydrochlorid und N-Hydroxysuccinimide. Weiterhin kommen heterobifunktionelle Reagenzien in Frage wie MBS (m-Maleimidobenzoyl-N-hydroxysuccinimidester) und andere.

Als zur Chemiluminszenz befähigte Gruppen kommen prinzipiell alle hierfür bekannten Substanzen in Frage wie sie beispielsweise in der DE—OS 29 21 781 ausführlich zusammengestellt sind. Weiterhin kommen in Frage, gewisse Acridiniumester, Oxalatester sowie das in der DE—OS 31 32 491 beschreibene Fluoresceinisothiocyanat.

Als Haptene kommen alle analytisch interessanten organischen Substanzen in Frage, die eine immunochemische Reaktion in einem Gasttier entwickeln, wenn sie in Form eines immunogenen Konjugats aus dem Hapten und einem Trägermolekül injiziert werden. Typische Haptene und die Gewinnung von hiergegen Spezifischen Antikörpen sind in der DE—OS 29 21 781, Seiten 45 bis 53 beschreiben.

In der Praxis bewährt hat sich als zur Chemilumineszenz befähigte Gruppe Luminol, welches in diazotierter Form als Diazoluminol an ein Polymeres gebunden werden kann, welches zur Diazokupplung geeignete Gruppen aufweist. Die Haptene werden vorzugsweise über eine Carbodiimid-Reaktion an das Polymere gebunden. Als Polymeres hat sich beispielweise das Glykoprotein Transferrin ausgezweichnet bewährt.

Der Antikörper gegen das jeweilige Hapten wird vorzugsweise dadurch gewonnen, daß man dieses Hapten mit einem anderen Carbodiimid an ein anderes Polymeres, beispielsweise Serum-Albumine koppelt und hiermit das zur Antikörperherstellung ausgewählte Tier behandelt.

Es hat sich gezeigt, daß derartige Antikörper hochspezifisch sind sowohl für das reine Hapten als auch für das erfindungsgemäße chemilumineszent-markierte Haptenkonjugat. Unspezifische Effekte und Kreuzreaktion werden hingengen hierbei vermieden.

Die Menge zur Chemilumineszenz befähigten Gruppen sowie die Menge des Haptens, die an das in der Verknüpfungsgruppe verwendete Polymere gebunden ist, sollten im allgemeinen mindestens 10 sein, so daß sich ein Molverhältnis Verknüpfungsgruppe zu zur Lumineszenz befähigten Gruppe zu Hapten von mindestens 1:10:10 ergibt.

Der für das Hapten spezifische Antikörper wird für den erfindungsgemäßen Lumineszenz Immunoassay vorzugsweise an einen festen Träger gebunden. Intensive Voruntersuchungen der Anmelderin haben zu dem Ergebnis geführt, daß als feste Träger Polystyrolkugeln besonders geeignet sind, die mit einem synthetischen Polypeptid wie p-Phe-Lys und Glutardialdehyd aktiviert wurden. Prinzipiell sind aber auch andere feste Trager geeignet, sofern der Antikörper hieran ohne nenneswerte Beeinträchtigung seiner spezifischen Reaktivität reproduzierbar und gleichmäßig gebunden werden kann.

Die Messung der Chemilumineszen von Luminol und Luminol-Derivaten kann an derartigen Kugeln in sehr einfacher Weise in einer Meßküvette erfolgen zu der in alkalischem Milieu Wasserstoffperoxid und Peroxidase zugegeben werden. Die dabei auftretende Lichtreaktion kann in bekannten Geräten, beispielsweise dem Luminometer LKB 1251 der Firma LKB, gemessen werden. Es hat sich gezeigt, daß die Handhabung, Empfindlichkeit und Präzision derartiger Lumineszenz Immunoassays für Haptene völlig vergleichbar ist mit der von entsprechenden Radioimmunoassays. Diese erfindungsgemäßen Lumineszenz Immunoassays sind obendrein für die Automation geeignet und können daher auch in Vollautomaten verwendet werden.

EP 0 135 071 B1

In den nachfolgenden Beispielen werden erfindungsgemäße chemilumineszent-markierte Haptenkonjugate sowie mit deren Hilfe hergestellte Lumineszenz Immunoassays für Haptene ausführlicher beschrieben, wobei die speziell gewählten zur Chemilumineszenz befähigten Gruppen, Verknüpfungsgruppen und Haptene keine Beschränkung der Erfindung bedeuten sollen.

## Beispiel 1

I. Herstellung eines chemilumineszent-markierten Haptenkonjugats z.B. ((Triiodothyronine ($T_3$))-Transferrin-(Daizoluminol)

a) Herstellung eines Transferrin-$T_3$-Konjugats mittels Carbodiimid-Kopplung:

44 mg Transferrin (human, Behring-Werke MW 88 000) 0,5 µmol) werden in 2 ml bidestilliertem Wasser gelöst. Nach rascher Zugabe von 42,9 mg (100 µmol) MCDI (1-Cyclohexyl-3-(2-morpholinyl-(4)-ethyl) carbodiimide-methyl-p-toluol-sulfonat) (Merck MW 429) gelöst in 500 µl bidestilliertem Wasser wird unter Rühren durch tropfenweise Zugabe von 0,01 N Salzsäure ein pH-Wert von 6 eingestellt. 17 mg Triiodothyronine ($T_3$) (MW 673, Sigma) werden in 1 ml bidestilliertem Wasser suspendiert und durch tropfenweise Zugabe von 0,5 mol/l NaOH bis zur vollständigen Lösung gerührt. Diese Lösung wird tropfenweise unter Rühren zu der obigen Lösungen gegeben, wobei durch Zugabe von 0,05 normaler Salzsäure der pH-Wert auf 6 gehalten wird. Man läßt anschließend 2 Stunden bei Raumtemperatur und 24 Stunden bei 3°C weiter reagieren. Die Lösung wird zentrifugiert. Das Transferrin-$T_3$-Konjugat im Überstand wird durch Gelfiltration über Sephadex® G—25 (Pharmacia) oder Ultrogel® A 6 (LKB) getrennt. Als Elutionspuffer wird 10 mM/l Phosphat pH 7—8 mit 0.15 mol/l Natriumchlorid verwendet. Die einzelnen Fraktionen werden photometrisch auf Proteingehalt sowie auf $T_3$-Immunoreaktivität getestet. Die Fraktionen, welche sowohl proteinhaltig als auch $T_3$-immunreaktiv sind, werden vereinigt. Aus den Meßwerten ergibt sich, daß das Substitutionsverhältnis $T_3$:Transferrin 14 mol/mol beträgt. Das Konjugat kann im gefrorenen Zustand gelagert oder lyophilisiert werden.

b) Kupplung von Diazoluminol an das Transferrin-$T_3$ Konjugat

I. Diazotierung von Luminol:

0,2 mmol Luminol (25,44 mg) (5-Amino-2,3-dihydro-1,4-phthalazindion, Ega-Chemie) werden in 5 ml 1 mol/l Salzsäure suspendiert und im Eisbad unter gleichmäßigem Rühren auf 0°C gekühlt. 1,5 mmol Kaliumnitrit (128 mg) werden in 1 ml Wasser gelöst und im Eisbad auf 0°C gekühlt. Die kalte Kaliumnitritlösung wird nun tropfenweise der Luminol-Suspension zugegeben, bis die gelbgrüne Trübung verschwunden ist und sich eine klare gelbe bis gelborange Lösung ausbildet. Überschüssige salpetrige Säure wird durch Zugabe einer Harnstofflösung entfernt. Die so erhaltene Diazoluminol-Lösung kann direkt verwendet werden. Sie ist im gefrorenen Zustand ca. eine Woche stabil.

Diazokupplung des Diazoluminols an das Transferrin-$T_3$-Konjugat:

20 mg lyophilisiertes Transferrin-$T_3$-Konjugat werden in 3 ml Wasser gelöst. Durch 0,1 mol/l Natriumcarbonat-Lösung wird der pH-Wert auf 9—9,5 eingestellt. Die Diazoluminol-Lösung wird tropfenweise bei 0°C unter Rühren zugegeben, und zwar bis zu einem Verhältnis von 100 Mol Diazoluminol pro Mol Transferrin-$T_3$-Konjugat. Hierbei wird mit weiterer Natriumcarbonat-Lösung der pH-Wert bei 9 gehalten. Das End-Reaktionsgemisch wird 24 Stunden bei 3°C gelagert. Das so erhaltene Transferrin-$T_3$-Diazoluminol-Konjugat wird in gleicher Weise wie das Transferrin-$T_3$-Konjugat durch Gelfiltration gereinigt. Die einzelnen Fraktionen werden getestet

1. mit einem Photometer bei 280 nm,
2. auf $T_3$-Immunoreaktivität,
3. auf Diazoluminol-Lumineszenz-Reaktion im LKB 1251 und
4. spezifische Bindung an $T_3$-Antikörper, sowie unspezifische Bindungseigenschaften.

Die Fraktionen mit meßbarem Proteingehalt, mit spezifischer Bindung an $T_3$-Antikörper jedoch ohne nennenswerte unspezifische Bindungseigenschaften werden vereinigt. Die Meßwerte ergeben ein Substitutionsverhältnis von Transferrin:$T_3$:Diazoluminol von 1:14:20. Das so erhaltene Konjugat kann sowohl lyophilisiert als auch im gefrorenen Zustand gelargert werden.

II. Verwendung des chemilumineszent-markierten Haptenkonjugats in einem Lumineszenz-Immunoassay für Haptene (Transferrin-$T_3$-Diazoluminol in einem $T_3$-Chemilumineszenzassay)
a) Immobilisierung von Antikörpern auf einem festen Träger ($T_3$ Antikörper auf Polystyrolkugeln)
Mit einem synthetischen Polypeptid (p-Phe-Lys, MW 30 000) beschichtete Polystyrolkugeln von 6,4 mm werden mit einer 0,5%-igen wäßrigen Lösung von Pentan-1,5-dial aktiviert. Die so erhaltenen Kugeln werden bei pH 7,5—8,5 in 0.05 mol/l Phosphatpuffer mit einem gereinigten $T_3$ Antikörper versetzt. Der $T_3$ Antikörper wurde gewonnen in Kaninchen mittels $T_3$-Rinderserumalbumin-Konjugat als Immunogen, das erhalten wurde unter Verwendung von 1-Ethyl-3-(-dimethyl-aminopropyl)carbodiimid (EDAC). Die gebildeten Schiff'schen Basen wurden in einigen Fällen vorsorglich mit Natriumborhydrid reduziert, jedoch zeigte sich kein wesentlicher Unterschied in der Stabilität. Die verbliebenen aktiven Gruppen wurden mit Rinderserumalbumin abgesättigt. Die Abstättigung war meist erst nach mehreren Tagen vollständig, was

4

durch Messung der unspezifischen Bindung kontrolliert wurde. Die mehrfach gewaschenen Kugeln wurden im Luftstrom getrocknet oder in 0,05 molarem Tris/Salzsäure-Puffer unter Zusatz von Rinderserumalbumin aufbewahrt. Getrocknete Kugeln wurden zur Rekonstitution vor dem Gebrauch eine Stunde in den gleichen Puffer gegeben.

b) Messungen der Chemilumineszenz

Als Oxidationssystem diente ein Gemisch aus Microperoxidase (Microperoxidase MP11-Sigma) 5 $\mu$mol/l), $H_2O_2$ (0,5%) und 0,8 mol/l NaOH. Als Puffer wurde ein 0,05 molarer Phosphatpuffer vom pH 7 unter Zusatz von 0,1 mol Natriumchlorid verwendet sowie 4 g/l Rinderserumalbumin und 0,015 mol/l $NaN_3$. Die Microperoxidase und der Puffer wurden kurz vor der Messung gemischt. Der pH-Wert der Endreaktionslösung betrug 13. Hierdurch erfolgt eine Verlangsamung der Chemilumineszenz-Kinetik, die Präzision wird jedoch gesteigert. Gemessen wurde nach dem Start der Reaktion durch $H_2O_2$-Zugabe, wobei die Lichtemission über eine Periode von 20 bis 30 Sekunden als Integral gemessen wird.

c) Durchführung des $T_3$-Lumineszenz-Immunoassays

Jeweils 100 µl Serumprobe bzw. Standard wurden mit 200 µl Inkubationspuffer (0,1 mol/l Tris-HCl von pH 7,4, 0,02 mol/l KCl, 0,2% Rinderserumalbumin) sowie 75 mg/100 ml ANS (8-Anilino-1-naphthalino-sulfonsäure) mit den mit Antikörpern beschichteten Polystyrolkugeln 2 Stunden bei Raumtemperatur inkubiert. Danach wurden 50 µl einer 1:100 Verdünnung des $T_3$-Transferrin-Diazoluminolkonjugats zugesetzt, kurz aufgeschüttelt und eine weitere Stunde bei Raumtemperatur inkubiert. Es wurde zweimal mit Inkubationspuffer und einmal mit 0,9%-iger Natriumchloridlösung gewaschen. Die Kugeln werden in die Meßküvetten überführt und wie oben beschrieben die Chemilumineszenz gemessen.

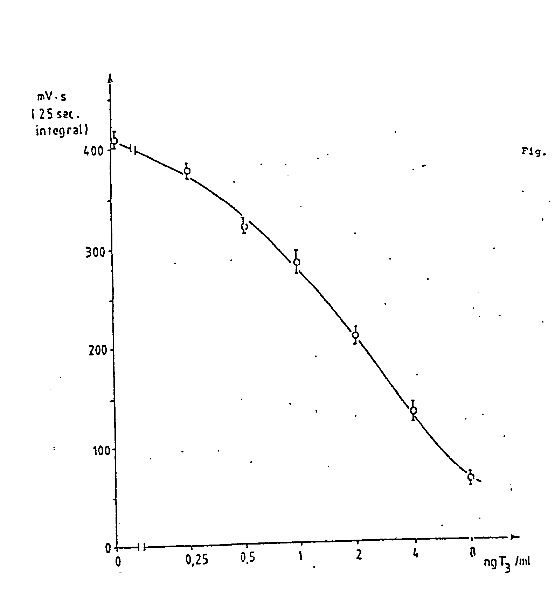

Typische Meßwerte sind in der Tabelle 1 (Figure 1) zusammengestellt. Die untere Nachweisgrenze liegt somit etwa bei 0,25 ng $T_3$/ml.

### TABELLE 1

| | $T_3$/Lumineszenzassay Chemilumineszenz (Integrationszeit = 20sec.) | | | $T_3$/RIA | | |
|---|---|---|---|---|---|---|
| ng $T_3$/ml | mV − s | MW | B/BO × 100 in % | IPM (a) (b) | MW | B/BO (%) |
| 0 | 415 429 | 422 | 100 | 15686 15151 | 15418 | 100 |
| 0,25 | 378 398 | 388 | 92 | 13937 13605 | 13771 | 89,3 |
| 0,50 | 324 331 | 327 | 78 | 12012 12195 | 12103 | 78,5 |
| 1 | 280 270 | 275 | 65 | 9828 9876 | 9852 | 63,9 |
| 2 | 199 210 | 204 | 48 | 7168 7194 | 7181 | 46,6 |
| 4 | 132 112 | 122 | 29 | 4981 5127 | 5054 | 32,8 |
| 8 | 66 52 | 59 | 14 | 2915 2967 | 2941 | 19,1 |

In der nachfolgenden Tabelle 2 sind erfindungsgemäß ermittelte Meßwerte, im Vergleich zu denen eines Radioimmunoassays, zusammengestellt.

# EP 0 135 071 B1

## TABELLE 2

Korrelation zwischen Meßwerten nach dem Chemilumineszenzassay und einem Radioimmunoassay unter Verwendung von drei Kontrollseren für $T_3$.

|  | $T_3$/RIA ng/ml | $T_3$/Lumineszenzassay ng/ml |
|---|---|---|
| Serum I | $0,34 \pm 0,2$ | $0,31 \pm 0,1$ *) |
| Serum II | $1,2 \pm 0,4$ | $0,97 \pm 0,4$ |
| Serum III | $3,4 \pm 1$ | $2,97 \pm 1,2$ |

*) Mittelwart ± dreifache Standardabweichung.
(Anzahl der jeweils gemessenen Proben n = 20)

### Beispiel 2

In analoger Weise wie in Beispiel 1 beschrieben wurde ein Proteinkonjugat wie folgt hergestellt: 385 mg Schweine-Thyreoglobulin mit ca. 80 Lysinresten wurde mit 1155 mg Bernsteinsäureanhydrid (11,6 mmol) umgesetzt. Dazu wurde das Thyreoglobulin unter Rühren in 25 ml Wasser gelöst und der pH-Wert mit einer 1 n NaOH auf 7 eingestellt. Das Bernsteinsäureanhydrid wurde in kleinen Portionen zugegeben und dabei der pH-Wert mit Hilfe von 1 n NaOH im Bereich von 7 bis 8 gehalten. Nach einer Stunde Rühren bei Raumtemperatur wurde der pH-Wert mit 1 n Salzsäure auf ca. 2,5 eingestellt, wobei sich ein dicker Niederschlag von ca. 80 ml bildete. Diese Suspension wurde in eine Amicon®-Druckfiltrationszelle überführt, auf 30 ml eingeengt und mehrmals mit 50 ml Wasser aufgeschlämmt und erneut eingeengt. Nach 6 Waschvorgängen war der pH-Wert 5. Der Inhalt der zelle wurde portioniert, eingefroren und lyophylisiert.

150 mg dieses Produktes wurden in 30 ml Wasser gelöst und unter gleichzeitiger Zugabe von 80 mg MCDI mit 38 mg L-$T_4$-Ethylester in 2 ml Methanol versetzt. Der pH-Wert wurde mit 1 n Salzsäure auf 4 eingestellt. Nach 30 Minuten wurde nochmals 80 mg MCDI zugegeben. Nach weiteren 20 Stunden Rühren bei Raumtemperatur wurde die Suspension in einen Dialyseschlauch überführt und 3 Tage lang dialysiert, wobei 2 mal täglich das Wasser gewechselt wurde. Der Dialyseschlauch wurde entleert und die Suspension zentrifugiert. Das klare Zentrifugat wurde in Portionen von 4 bis 5 ml unterteilt, eingefroren und lyophylisiert. Der Niederschlag wurde in Ammoniak oder Ammoniumacetat gelöst, danach ebenfalls portioniert, eingefroren und gefriergetrocknet. Die weitere Aufarbeitung und Kupplung mit Diazoluminol erfolgte in analoger Weise wie in Beispiel 1 beschrieben, wobei ein $T_4$-Lumineszenz-Immunoassay entstand.

## Patentansprüche

1. Lumineszenz Immunoassay für Haptene bestehend aus
A) einem für das jeweilige Hapten spezifischen Antikörper und
B) einem chemilumineszent markiertem Haptenkonjugat, enthaltend
a) eine zur Chemilumineszenz befähigte Gruppe,
b) eine Verknüpfungsgruppe und
c) ein Hapten, dadurch gekennzeichnet, daß die Verknüpfungsgruppe (b) ein kettenförmiges Polymeres mit wiederkehrenden funktionellen Gruppen ist, an welches pro Mol sowohl mehrere Mole zur Lumineszenz befähigte Gruppen als auch mehrere Mole Hapten gebunden sind.

2. Lumineszenz Immunoassay gemäß Anspruch 1, dadurch gekennzeichnet, daß der in A) benutzte spezifische Antikörper unter Verwendung eines Haptens als Immunogen gewonnen wurde, welches an eine andere Verknüpfungsgruppe gebunden ist als die gemäß b) verwendete.

3. Lumineszenz Immunoassay gemäß Anspruch 1 oder 2, dadurch gekennzeichnet, daß die Verknüpfungsgruppe b) ein Peptid, ein Glykoprotein, ein Glykolipid oder ein Kohlenhydrat ist.

4. Lumineszenz Immunoassay gemäß einem der Ansprüche 1 bis 3, dadurch gekennzeichnet, daß das Molverhältnis der Verknüpfungsgruppe b) zu der chemilumineszenz befähigten Gruppe a) zum Hapten c) mindestens 1:10:10 beträgt.

5. Lumineszenz Immunoassay gamäß einem der Ansprüche 1 bis 4, dadurch gekennzeichnet, daß der für das jeweilige Hapten spezifische Antikörper A) auf einem festen Träger gebunden vorliegt.

6. Chemilumineszent-markiertes Haptenkonjugat, enthaltend
a) eine zur Chemilumineszenz befähigte Gruppe,
b) eine Verknüpfungsgruppe und
c) ein Hapten, dadurch gekennzeichnet, daß die Verknüpfungsgruppe b) ein kettenförmiges Polymeres mit wiederkehrenden funktionellen Gruppen ist, an welches pro Mol sowohl mehrere Mole zur Lumineszenz befähigte Gruppen als auch mehrere Mole Hapten gebunden sind.

6

# EP 0 135 071 B1

7. Markiertes Haptenkonjugat gemäß Anspruch 6, dadurch gekennzeichnet, daß die Verknüpfungsgruppe

b) ein Peptid, ein Glykoprotein, ein Glykolipid oder ein Kohlenhydrat ist.

8. Markiertes Haptenkonjugat gemäß Anspruch 6 oder 7, dadurch gekennzeichnet, daß das Molverhältnis der Verknüpfungsgruppe b) zu der zur Lumineszenz befähigten Gruppe a) zum Hapten c) mindestens 1:10:10 beträgt.

9. Verfahren zur Herstellung eines lumineszentmarkierten Haptenkonjugats, enthaltend a) eine zur Chemilumineszent befähigte Gruppe,

b) eine Verknüpfungsgruppe und

c) ein Hapten, dadurch gekennzeichnet, daß man ein kettenförmiges Polymeres mit wiederkehrenden funktionellen Gruppen b) gleichzeitig oder nacheinander in beliebiger Reihenfolge sowohl mit mehreren Molen zur Lumineszenz befähigter Gruppen a) als auch mit mehreren Molen Hapten c) chemisch verbindet.

## Revendications

1. Produit de dosage immunologique par luminescence d'haptènes constitué par

A) un anticorps spécifique pour l'haptène considéré et

B) un produit de conjugaison chimiluminescent d'haptènes contenant

a) un groupe capable de chimiluminescence

b) un groupe de couplage, et

c) un haptène

caractérisé en ce que le groupe de couplage b) est un polymère aliphatique avec des groupes fonctionnnels répétes, avec lequel sont combinées par mole plusieurs moles de groups capables de luminescence ainsi que plusieurs moles d'haptène.

2. Produite de dosage immunologique par luminescene selon la revendication 1, caractérisé en ce que l'anticorps spécifique employé en A) a été obtenu en utilisant comme immunogène un haptène qui est fixé sur un groupe de couplage autre que celui qui est utilisé d'après b).

3. Produit de dosage immunologique par luminescence d'après la revendication 1 ou 2, caractérisé en ce que le groupe de couplage b) est un peptid, une glycoprotéine, un glycolipide ou un glucide.

4. Produit de dosage immunologique par luminescence selon l'une des revendications 1 à 3, caractérisé en ce que le rapport molaire du groupe de couplage b) au groupe capable de chimiluminescence a) et à l'haptène c) est d'au moins 1:10:10.

5. Produit de dosage immunologique par luminescence selon l'une des revendications 1 à 4, caractérisé en ce que l'anticorps A) spécifique pour l'haptène considéré est fixé sur un support solide.

6. Produit de conjugaison marqué chimiluminescent d'haptène consisuté par:

a) un groupe capable de chimiluminescence,

b) un groupe de couplage et

c) un haptène,

caractérisé en ce que le groupe de couplage b) est un polymère aliphatique avec des groupes foncitionnels répétes, avec lequel sont combinées par mole plusieurs moles de groups capables de luminescence ainsi que plusieurs moles d'haptène.

7. Produit de conjugaison d'haptène marqué selon la revendication 6, caractérisé en ce que le groupe de couplage (b) est: un protide, une glycoprotéine, un glycolipide ou un glucide.

8. Produit de conjugaison d'haptène marqué selon la revendication 6 ou 7, caractérisé en ce que le rapport molaire du groupe de couplage b) au groupe capable de luminescence a) et à l'haptène c) est d'au moins 1:10:10.

9. Procédé de préparation d'un produit de conjugaison d'haptène marqué luminescent comprenant

a) un groupe capable de chimiluminescence,

b) un groupe de couplage et

c) un haptène, caractérisé en ce qu'on combine chimiquement un polymère aliphatique avec des groupes fonctionnels b) répétés, simultanément ou successivement dans un ordre facultatif, ainsi qu'avec plusieurs moles de groups a) capables de luminescence et avec plusieurs moles d'haptène c).

## Claims

1. A luminescence immunoassay for haptens consisting of

(A) an antibody specific for the respective hapten and

(B) a chemiluminescent-labelled hapten conjugate containing

(a) a group capable of chemiluminescence,

(b) a linkage group, and

(c) a hapten, characterized in that the linkage group (b) is a chain-like polymer having repeating functional groups and has bound thereto, per mole, several moles of groups capable of luminescence and several moles of haptens.

7

2. The luminescence immunoassay according to claim 1, characterized in that the specific antibody used in (A) was prepared using as the immunogen a hapten bound to a linkage group other than the linkage group used according to (b).

3. The luminescence immunoassay according to claims 1 or 2, characterized in that the linkage group (b) is a peptide, a glycoprotein, a glycolipid or a carbohydrate.

4. The luminescence immunoassay according to any of claims 1 to 3, characterized in that the molar ratio of the linkage group (b) to the group (a) capable of chemiluminescence to the hapten (c) is at least 1:10:10.

5. The luminescence immunoassay according to any of claims 1 to 4, characterized in that the antibody (A) which is specific for the respective hapten is present bound to a solid supporting material.

6. A chemiluminescent-labelled hapten conjugate containing

(a) a group capable of chemiluminescence,

(b) a linakge group, and

(c) a hapten, characterized in that the linkage group (b) is a chain like polymer having repeating functional groups and has bound thereto, per mole, several moles of groups capable of luminescence and several moles of haptens.

7. The labelled hapten conjugate according to claim 6, characterized in that the linkage group (b) is a peptide, a glycoprotein, a glycolipid or a carbohydrate.

8. The labelled hapten conjugate according to claims 6 or 7, characterized in that the molar ratio of the linkage group (b) to the group (a) capable of chemiluminescence to the hapten (c) is at least 1:10:10.

9. A method for the preparation of a chemiluminescent-labelled hapten conjugate containing

(a) a group capable of chemiluminescence,

(b) a linkage group, and

(c) a hapten, characterized in that a chain-like polymer having repeating functional groups (b) is allowed to be chemically bonded, simultaneously or subsequently in any sequence, to several moles of groups (a) and to several moles of hapten (c).

EP 0 135 071 B1

Fig. 1